(19) Europäisches Patentamt
European Patent Office
Office européen des brevets

(11) Veröffentlichungsnummer : **0 024 286**
**B1**

(12) # EUROPÄISCHE PATENTSCHRIFT

(45) Veröffentlichungstag der Patentschrift : **22.09.82**

(51) Int. Cl.³ : **C 07 C 51/60**, C 07 C 63/24, C 07 C 63/30, B 01 J 31/02

(21) Anmeldenummer : **80103482.8**

(22) Anmeldetag : **21.06.80**

(54) **Verfahren zur Herstellung aromatischer Dicarbonsäuredichloride und ihre Verwendung bei der Herstellung von Polykondensaten.**

(30) Priorität : **16.08.79 DE 2933148**

(43) Veröffentlichungstag der Anmeldung : **04.03.81 (Patentblatt 81/09)**

(45) Bekanntmachung des Hinweises auf die Patenterteilung : **22.09.82 Patentblatt 82/38**

(84) Benannte Vertragsstaaten : **BE CH DE FR GB IT LI NL**

(56) Entgegenhaltungen :
**DE A 2 525 442**
**GB A 970 072**
**GB A 2 004 881**

(73) Patentinhaber : **BAYER AG**
**Zentralbereich Patente, Marken und Lizenzen**
**D-5090 Leverkusen 1, Bayerwerk (DE)**

(72) Erfinder : **Freitag, Dieter, Dr.**
**Hasenheide 10**
**D-4150 Krefeld (DE)**
Erfinder : **Schmidt, Manfred, Dr.**
**1020 North-Third-Street**
**New Martinsville 26155 (US)**
Erfinder : **Bottenbruch, Ludwig, Dr.**
**Woehlerstrasse 5**
**D-4150 Krefeld (DE)**

**EP 0 024 286 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung aromatische Dicarbonsäuredichloride und ihre Verwendung bei der Herstellung von Polykondensaten.

Die Erfindung betrifft ein Einstufen-Verfahren zur Herstellung von hochreinen polykondensationsfähigen aromatischen Dicarbonsäuredichloriden.

Die Erzeugung von aliphatischen und aromatischen Säurechloriden durch Reaktion einer Carbonsäure mit Phosgen ist in den US-PS 3 184 506, 3 544 626, 3 544 627, 3 547 960 sowie in den DE-OS 2 400 007 und 2 321 122 beschrieben. Nach diesen Verfahren werden als Reaktionsprodukte dunkelgefärbte Carbonsäurechloride in einer Reinheit von 96 bis 99 % erhalten. Aromatische Dicarbonsäurechloride dieses geringen Reinheitsgrades können nicht direkt nach dem Zweiphasengrenzflächenpolykondensationsverfahren zur Herstellung von hochmolekularen Polykondensaten, wie aromatischen Polyamiden oder aromatischen Polyestern, eingesetzt werden. Ihr Gehalt an nicht oder nur halbseitig umgesetzten Dicarbonsäuren stört die Polykondensation, verursacht unerwünschten Kettenabbruch, und ergibt Polymerisate mit endständigen Carboxylgruppen. Die so hergestellten aromatischen Dicarbonsäuredichloride sind durch Verunreinigungen dunkel gefärbt und enthalten durch Umsetzung mit den Katalysatoren entstandene störende Carbamidsäurechloride (vgl. Chem. Ref. 1973, Vol. 73, Nr. 1, Seite 77 oder Angewandte Chemie (1974), Jahrg. 1962, Nr. 21, Seite 864).

Um farblose Dicarbonsäuredichloride zu erhalten, müssen die Rohprodukte durch Umkristallisation oder Destillation gereinigt werden. Dies erfordert zusätzlichen Aufwand und vermindert die Ausbeute ; bei aromatischen Dicarbonsäuredichloriden besteht die Gefahr einer spontanen Zersetzung.

Gegenstand der Erfindung ist ein Einstufen-Verfahren zur Herstellung von reinen aromatischen Dicarbonsäuredichloriden durch Umsetzung aromatischer Dicarbonsäuren mit Phosgen in Anwesenheit eines Katalysators und gegebenenfalls in einem Lösungsmittel und/oder Verdünnungsmittel, das dadurch gekennzeichnet ist, daß man als Katalysatoren tertiäre Phosphine verwendet. Die so erhaltenen aromatischen Dicarbonsäuredichloride sind praktisch farblos und enthalten neben den eingesetzten Katalysatoren 0,1 % Verunreinigungen oder weniger, so daß sie ohne Nachreinigung zur Herstellung von farblosen, hochmolekularen Polykondensaten eingesetzt werden können.

Die erfindungsgemäß als Katalysatoren eingesetzten tertiären Phosphine stören beispielsweise die Herstellung von aromatischen Polyestern nicht. Die verwendeten Katalysatoren können nach Beendigung der Reaktion entweder einfach durch Andestillieren des Reaktionsgemisches aus diesem entfernt werden, jedoch stören verbleibende Reste des Katalysators im erhaltenen Dicarbonsäuredichlorid die Umsetzung dieser Dicarbonsäuredichloride zu beispielsweise aromatischen Polyestern nach dem Verfahren der Zweiphasengrenzflächenreaktion nicht, da sie gleichzeitig wirksame Umesterungskatalysatoren für diese Polykondensationsreaktion darstellen.

Als erfindungsgemäß wirksame Katalysatoren sind tertiäre Phosphine der allgemeinen Struktur (I) geeignet.

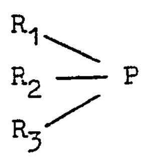

(I)

wobei

$R_1$, $R_2$, $R_3$ gleich oder verschieden und $C_1$-$C_8$-Alkyl, $C_6$-$C_{10}$-Aryl, $C_7$-$C_{20}$-Alkylaryl oder Arylalkyl sein können. Bevorzugt sind $R_1$, $R_2$ und $R_3$ $C_6$-$C_{10}$-Arylreste, wie Phenyl oder mit $C_1$-$C_4$-Alkylresten substituiertes Phenyl.

Geeignete Katalysatoren sind :

Tribenzylphosphin, Triisopropylphosphin und Tributylphosphin.

Besonders geeignet ist Triphenylphosphin.

Erfindungsgemäß werden 0,1 bis 3,0 Gew.-%, vorzugsweise 0,2 bis 1,5 Gew.-%, bezogen auf die eingesetzten aromatischen Dicarbonsäuren, an tertiären Phosphinen der allgemeinen Struktur (I) eingesetzt.

Aromatische Dicarbonsäuren entsprechen den folgenden Formeln

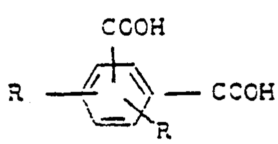

(II)

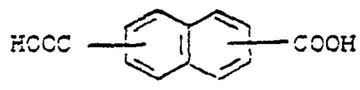

(III)

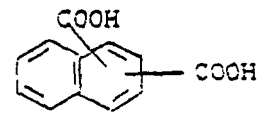

(IV)

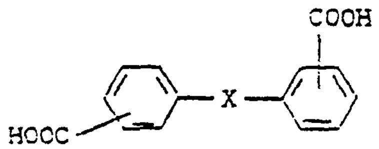

(V)

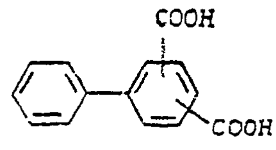

(VI)

mit

R = H, $C_1$-$C_4$-Alkyl oder Halogen (bevorzugt Chlor und Brom),

X = einfache Bindung, —O—, —S—, —$CH_2$, $-\underset{CH_3}{\overset{CH_3}{C}}-$, $C_5$-$C_7$-Cycloalkylen.

Auch Gemische können verwendet werden.

Beispielhaft seien genannt :

Phthalsäure, Isophthalsäure, Terephthalsäure, Gemische aus Iso- und Terephthalsäure, Diphensäure und 1,4-Naphthalindicarbonsäure.

Als Lösungs- oder Verdünnungsmittel werden vorzugsweise das oder die (bei Gemischen) aromatischen Dicarbonsäuredichloride eingesetzt, die während der Reaktion gebildet werden. Ebenso ist Benzoylchlorid als Lösungsmittel geeignet. Inerte Verdünnungsmittel, wie aliphatische oder aromatische Kohlenwasserstoffe, Halogen-substituierte aromatische Kohlenwasserstoffe, Halogen-substituierte aliphatische Kohlenwasserstoffe oder gesättigte aliphatische Ester, können ebenfalls verwendet oder mitverwendet werden. Die Reaktionstemperatur ist im allgemeinen 70 bis 200 °C, vorzugsweise 100 bis 180 °C.

Zur Durchführung des erfindungsgemäßen Verfahrens in Gegenwart aromatischer Dicarbonsäuredichloride können die aromatischen Dicarbonsäuren nach Zugabe der erfindungsgemäßen Katalysatoren in 8 bis 80 Gew.-%, bezogen auf das gesamte Reaktionsgemisch, der entsprechenden, bereits früher hergestellten aromatischen Dicarbonsäuredichloride bei 140 bis 180 °C suspendiert oder gelöst und anschließend bei 70 bis 200 °C, vorzugsweise bei 100 bis 180 °C, mit Phosgen umgesetzt werden.

Das Mol-Verhältnis aromatische Dicarbonsäure zu Phosgen ist bevorzugt 1 : 2 bis 1 : 2, 5, d.h., ein geringer Überschuß an Phosgen ist ratsam, um Verluste zu ersetzen, die bei der Austreibung von $CO_2$- und HCL-Gas aus dem Reaktionsgemisch während der Phosgenierung entstehen.

Das erfindungsgemäße Verfahren kann diskontinuierlich oder kontinuierlich ausgeführt werden. In einer kontinuierlichen Ausführungsform läßt man in einem Reaktionsrohr eine 120-180 °C heiße Lösung aus aromatischer Dicarbonsäure, Dicarbonsäuredichlorid und Katalysator nach unten gegen aufströmendes Phosgengas strömen und nimmt am Boden des Reaktionsrohres aromatisches Dicarbonsäuredichlorid und Katalysator ab.

In einer diskontinuierlichen Ausführungsform werden unter Normaldruck, bei höherer Temperatur gegebenenfalls unter Druck, aromatische Dicarbonsäure, aromatisches Dicarbonsäuredichlorid und Katalysator vorgelegt. Dann wird unter Rühren auf 140 bis 180 °C erhitzt, wobei sich die aromatische Dicarbonsäure unter HCl-Entwicklung und Anhydridbildung ganz oder teilweise löst. Bei dieser Temperatur werden pro Mol aromatischer Dicarbonsäure 2 bis 2,5 Mol gasförmiges Phosgen eingeleitet.

Nach Entfernen von überschüssigem Phosgen, HCl- und $CO_2$-Gas durch kurzzeitiges Anlegen von Vakuum erhält man einen Rückstand, der neben der Menge an eingesetztem Katalysator zu ⩾ 99,9 % aus aromatischem Dicarbonsäuredichlorid besteht, und der ohne Nachreinigung zu hochmolekularen, farblosen Polykondensaten umgesetzt werden kann.

## Beispiel 1

In einem 250 ml Kolben mit Magnetrührer, Thermometer, Gaseinleitungsfritte und 2 übereinandergesteckten Intensivkühlern (−10° bis −20 °C) werden vorgelegt und aufgeheizt :

66,4 g Isophthalsäuredichlorid,
66,4 g Terephthalsäuredichlorid,
16,6 g (0,1 Mol) Isophthalsäure,
16,6 g (0,1 Mol) Terephthalsäure,
0,166 g Triphenylphosphin (≙ 0,5 Gew.-%, bezogen auf Säuren)

Die blaßgelbe Suspension (> 50 °C) wird innerhalb von 30 Min. auf 178 °C aufgeheizt, wobei HCl-Entwicklung auftritt. Nach 1 h bei 178-181 °C läßt die bis dahin zügige HCl-Entwicklung nach, und der Ansatz ist bis auf eine kleine Restmenge an Säuren in Lösung.

Anschließend wird noch 30 Min. Stickstoff durch das Reaktionsgemisch geleitet. Hierbei geht die Restmenge an Säuren beschleunigt in Lösung.

In diese klare, hellgelbe Lösung wird innerhalb von 45 Min. bei 180-184 °C Phosgen eingeleitet. Dabei entweicht über die Kühler $CO_2$, das Spuren Phosgen mitreißt. Diese Spuren werden in einer Kühlfalle dem Gemisch entzogen. Zur Sicherheit wird hinter die Kühlfalle ein mit Aktiv-Kohle gefüllter Turm geschaltet, um Restspuren Phosgen zu vernichten. Nach 45 Min. Phosgen-Einleitung tritt bei klarer Lösung ein Temperaturabfall von 184 °C auf 176 °C ein, bedingt durch den Phosgenrückfluß. Die Phosgenierung wird beendet. Zur Beseitigung des Phosgenüberschusses wird die Kühlung abgeschaltet, mit $N_2$ gespült und bei 148 °C und unter vermindertem Druck 10 Min. andestilliert.

Der Rückstand enthält ≥ 99,9 % Säurechloride,
< 0,1 % COOH,
< 0,05 % HCl.

Mit diesen Säurechloriden und mit Bisphenol A wurde in Gegenwart von 3 Mol-% tert.-Butylphenol als Kettenabbrecher nach dem üblichen Grenzflächenpolykondensationsverfahren ein aromatischer Polyester mit einer $\eta$ rel = 1.291 hergestellt.

Beispiel 2

203 g (1 Mol) Isophthalsäuredichlorid, 166 g Isophthalsäure (1 Mol) und 0,8 g Triphenylphosphin werden im Rundkolben, der mit Thermometer, Rührer und einem durch Kühlsole auf −20 °C gehaltenen Intensivkühler ausgestattet ist, auf 148 °C erhitzt. Bei 148-157 °C wird unter Rühren und Rückflußsieden so lange Phosgen eingeleitet, bis im Reaktionsgemisch ein Temperaturabfall auf 146 °C eintritt.

Nach Abkühlen auf 120 °C wird Wasserstrahlvakuum angelegt, wodurch überschüssiges Phosgen sowie im Reaktionsgemisch gelöstes HCl- und $CO_2$-Gas entfernt werden.

Ausbeute : 406,2 g eines farblosen Rückstandes, der aus 0,3 g des eingesetzten Katalysators und 405,9 g eines 100 %igen Isophthalsäuredichlorids (titrimetrisch bestimmt) besteht.

Beispiel 3

101,5 g (0,5 Mol) Isophthalsäuredichlorid, 101,5 g (0,5 Mol) Terephthalsäure-dichlorid, 83 g (0,5 Mol) Isophthalsäure, 83 g (0,5 Mol) Terephthalsäure und 0,8 g Triphenylphosphin werden, wie im Beispiel 1 beschrieben, mit Phosgen umgesetzt.

Ausbeute : 406,3 g eines farblosen Rückstandes, der zu 0,3 g aus eingesetztem Katalysator und zu 406 g eines Gemisches aus 100 %igem Iso- und Terephthalsäuredichlorid (titrimetrisch bestimmt) besteht.

Beispiel 4

101,5 g (0,5 Mol) Isophthalsäuredichlorid, 101,5 g (0,5 Mol) Terephthalsäuredichlorid, 83 g (0,5 Mol) Isophthalsäure, 83 g (0,5 Mol) Terephthalsäure und 0,8 g Tributylphosphin werden, wie im Beispiel 1 beschrieben, mit Phosgen umgesetzt.

Ausbeute: 406,4 g 99,9 %iges Iso- und Terephthalsäuredichlorid, das 0,4 g des eingesetzten Katalysators enthält.

Beispiel 5

101,5 g (0,5 Mol) Isophthalsäuredichlorid, 101,5 g (0,5 Mol) Terephthalsäuredichlorid, 83 g (0,5 Mol) Isophthalsäure, 83 g (0,5 Mol) Terephthalsäure und 0,6 g Tribenzylphosphin werden, wie im Beispiel 1 beschrieben, mit Phosgen umgesetzt.

Ausbeute : 406,4 g eines nahezu farblosen Rückstandes aus 99,9 %igem Iso- und Terephthalsäuredichlorid (titrimetrisch bestimmt) und 0,4 g des eingesetzten Katalysators bestehend.

**Ansprüche**

1. Einstufen-Verfahren zur Herstellung von reinen, aromatischen Dicarbonsäuredichloriden durch Umsetzung einer aromatischen Dicarbonsäure oder eines aromatischen Dicarbonsäuregemisches mit Phosgen in Anwesenheit eines Katalysators, und gegebenenfalls in einem Lösungs- oder Verdünnungsmittel, dadurch gekennzeichnet, daß man als Katalysatoren tertiäre Phosphine verwendet.

2. Verfahren nach Anspruch 1, dadurch gekennzeichnet, daß der Katalysator eine Verbindung der Formel I

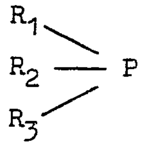

(I)

ist, worin

$R_1$, $R_2$ und $R_3$ $C_6$-$C_{10}$-Arylreste darstellen.

3. Verwendung der gemäß Anspruch 1 erhaltenen aromatischen Dicarbonsäuredichloride zur Herstellung von Polykondensaten.

**Claims**

1. A one-step process for the preparation of pure aromatic dicarboxylic acid dichlorides by reacting an aromatic dicarboxylic acid or an aromatic dicarboxylic acid mixture with phosgene in the presence of a catalyst and, optionally, in a solvent or diluent, characterised in that the catalysts used are tertiary phosphines.

2. A process according to Claim 1, characterised in that the catalyst is a compound of the formula I

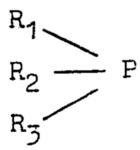

(I)

wherein

$R_1$, $R_2$, and $R_3$ represent $C_6$-$C_{10}$-aryl radicals.

3. The use of the aromatic dicarboxylic acid dichlorides obtained according to Claim 1 for the preparation of polycondensates.

**Revendications**

1. Procédé en un stade pour la fabrication de dichlorures d'acides dicarboxyliques aromatiques purs par réaction d'un acide dicarboxylique aromatique ou d'un mélange d'acides dicarboxyliques aromatiques avec du phosgène en présence d'un catalyseur et éventuellement dans un solvant ou diluant, caractérisé en ce qu'on utilise des phosphines tertiaires comme catalyseurs.

2. Procédé selon la revendication 1, caractérisé en ce que le catalyseur est un composé de formule I :

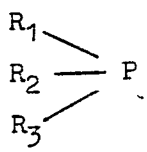

(I)

dans laquelle

$R_1$, $R_2$ et $R_3$ représentent des radicaux aryle en $C_6$-$C_{10}$.

3. Utilisation des dichlorures d'acides dicarboxyliques aromatiques obtenus selon la revendication 1 pour la fabrication de polycondensats.